# EUROPEAN PATENT APPLICATION

(11) **EP 3 081 150 A1**
(43) Date of publication of application: **19.10.2016**
(21) Application number: 15163751.9
(22) Date of filing: 15.04.2015
(51) Int. Cl.: A61B 5/00

(54) **SKIN PROTRUSION SIZE DETERMINATION**

(71) Applicant: Gant, Alasdair Ivan Andre, London N1 0GJ (GB); Story, Alistair Langfield, London N1 0GJ (GB)
(72) Inventor: Gant, Alasdair Ivan Andre, London N1 0GJ (GB); Story, Alistair Langfield, London N1 0GJ (GB)
(74) Representative: Zijlstra, Robert Wiebo Johan

(57) **Abstract**

Disclosed is a computer program product comprising a computer-readable medium carrying program code for, when executed on a processor (110) of a device (100) further comprising a touchscreen (120), implementing the steps of determining a touchscreen stimulus generated by a skin protrusion (20) in a skin region (10) brought into contact with the touchscreen; processing the touchscreen stimulus to derive a size of said skin protrusion from the touchscreen stimulus; and generating a signal indicative of said derived size. Further disclosed is a device comprising such a computer program product, a diagnostic test kit including the computer program product and a computer-implemented method.

## Description

### FIELD OF THE INVENTION

The present invention relates to a computer program product for determining the size of a skin protrusion.

The present invention further relates to a device comprising such a computer program product.

The present invention yet further relates to a diagnostic test kit including such a computer program product.

The present invention still further relates to a method of determining the size of a skin protrusion.

### BACKGROUND OF THE INVENTION

Medical care plays a central role in modern society. Many diagnostic methods and curative treatments are now routinely available to combat many serious, e.g. potentially life-threatening, diseases, which has dramatically improved both quality of life as well as life expectancy. Medical practitioners play a pivotal role in facilitating access to such medical care by assessing medical conditions when meeting a patient and administering the correct medical treatment, e.g. a course of medication, a surgical procedure and so on, to treat the diagnosed medical condition.

However, it is not always convenient or even possible for the patient to visit a medical practitioner. For example, the patient may have a busy schedule that does not allow for the patient to visit the medical practitioner several times in a short period of time, or the patient may live in a remote location without a medical practitioner in the vicinity. The latter is for instance is particularly likely in developing regions of the world, e.g. Africa, India, and so on.

A patient for instance may need to see the medical practitioner several times when undergoing diagnostic testing, e.g. to administer a diagnostic test during an initial visit and to collect and interpret the diagnostic test results during a subsequent visit. An example of such a diagnostic test is a skin test for tuberculosis, in which the patient is percutaneously administered with tuberculosis antigens after which the test must develop over a certain period of time, e.g. 2-3 days, before the medical practitioner can evaluate the test results to reach the diagnosis and prescribe an appropriate treatment. The inconvenience or logistic complexity associated with such visits can discourage or even prevent a patient from seeking medical treatment, thus leaving potentially life-threatening and/or contagious diseases undiagnosed.

### SUMMARY OF THE INVENTION

The present invention seeks to provide a computer program product that can generate information of intermediate diagnostic relevance by evaluation of a skin protrusion.

The present invention further seeks to provide a device comprising such a computer program product.

The present invention still further seeks to provide a diagnostic test kit product including such a computer program product.

The present invention yet further seeks to provide a computer-implemented method of generating information of intermediate diagnostic relevance by evaluation of a skin protrusion.

According to an aspect, there is provided a computer program product comprising a computer-readable medium carrying program code for, when executed on a processor of a device further comprising a touchscreen, implementing the steps of determining a touchscreen stimulus generated by a skin protrusion in a skin region brought into contact with the touchscreen; processing the touchscreen stimulus to derive a size of said skin protrusion from the touchscreen stimulus and generating a signal indicative of the derived size. The present invention is based on the insight that skin protrusions, i.e. skin bumps, may be evaluated using touchscreen technology, which therefore facilitates the remote or even automatic evaluation of a skin condition or a diagnostic test result such that a medical practitioner is not required to perform the evaluation. This therefore obviates the need for a patient to visit the medical practitioner to obtain the test result or diagnosis. In particular, as touchscreen technology is ubiquitous in modern society, e.g. virtually all smart devices such as smart phones, tablets, personal digital assistants and the like rely on touchscreen technology for their primary user interface, this computer program product therefore facilitates the generation of skin-related information of intermediate diagnostic relevance or even skin-based diagnosis in remote locations without immediate medical assistance, such as the aforementioned developing regions of the world. This therefore significantly reduces the risk that serious illnesses remain undiagnosed.

In an embodiment, said determining comprises determining at least one of an area of the touchscreen in contact with the skin protrusion and a pressure applied to the touchscreen by said skin protrusion. This data may be used to determine the size of the skin protrusion in a straightforward manner, e.g. using appropriate algorithms.

The skin protrusion may be a reaction to a diagnostic test applied to the skin region, in which case the program code may further implement the step of comparing the size of the skin protrusion against a predefined threshold to determine a test result. An example of such a diagnostic test in which a patient's skin is used as a laboratory is a tuberculosis test, where the amount of reaction of the skin to tuberculosis antigens injected into the skin is indicative of the patient being infected with tuberculosis.

The device further may comprise a camera under control of said processor, the program code further implementing the step of capturing an image of the skin protrusion and extracting colorimetric information from the captured image to determine the test result. A combination of skin protrusion size and colorimetric characterization (e.g. to assess erythema) may yield particularly reliable diagnostic results, e.g. in the case of a tuberculosis test.

The device may further comprise a camera under control of said processor, the program code further implementing the step of capturing an identifier of a test kit for administering the diagnostic test with said camera. This for instance allows for identification of the type of test, which identification may be used for a variety of purposes such as (generating and) providing the user with test administration instructions, providing the user with test result collection instructions or providing a remote clinician with details about the test administered by the user by transmission of the signal to a remote component, e.g. a remote device.

The step of capturing said identifier may be performed when administering the diagnostic test to the skin region, wherein the program code may further implement the steps of determining a time period between capturing the identifier and determining the touchscreen stimulus; and determining compliance with a recommended test regime from the determined time period. This for instance may involve checking if the user is attempting to collect the test result at the correct point in time, i.e. after sufficient development of the test, or may involve monitoring the time elapsed after administering the test and advising the user when to collect the test result.

In a preferred embodiment, the device further comprises a wireless communication unit under control of said processor, the program code further implementing the step of transmitting said signal to a remote recipient with the wireless communication unit. Said signal may include the diagnostic test result and the test identifier in case of the skin protrusion being the result of an administered diagnostic test such as a tuberculosis test. This may be used to provide a remote medical practitioner with the information of intermediate diagnostic relevance or a diagnosis, thereby facilitating the medical practitioner to take further actions, e.g. reach a diagnosis and/or issue medical treatment, e.g. a course of medication, to the user of the computer program product.

The device may further comprise a global positioning unit under control of said processor, the program code further implementing the step of determining a global position of the device with the global positioning unit upon determining the touchscreen stimulus and including the determined global position in the signal. This for instance informs the remote medical practitioner of the location of the user of the computer program product, such that the medical practitioner can direct follow-up actions to the detected global position, in case the location of the user is unknown.

According to another aspect, there is provided a device comprising a touchscreen, a processor configured to interpret touchscreen contact information and the computer program product of any of the above embodiments, wherein the processor is adapted to execute said program code. Such a device may be a mobile communications device such as a smart phone, a tablet, a digital personal assistant and so on.

According to yet another aspect, there is provided a diagnostic test kit including a diagnostic test delivery device and the computer program product of any of the above embodiments. Such a diagnostic test kit facilitates a user to perform a diagnostic test without the presence of a medical practitioner, thereby making such diagnostic tests available to users unwilling or incapable of visiting a medical practitioner for such a test.

In an embodiment, the diagnostic test delivery device comprises a micro-needle device containing tuberculosis antigens for delivery to a skin region of a user. Such a tuberculosis test kit product makes it possible to remotely diagnose tuberculosis, which may aid in combatting this disease, e.g. in inaccessible locations such as remote regions of developing parts of the world.

According to a further aspect, there is provided a computer-implemented method for execution by a processor of a device further comprising a touchscreen, the method comprising determining a touchscreen stimulus generated by a skin protrusion in a skin region brought into contact with the touchscreen; processing the touchscreen stimulus to derive a size of said skin protrusion from the touchscreen stimulus and generating a signal indicative of the derived size.

The method may further comprise deriving a result of intermediate diagnostic relevance from the derived size, wherein the signal is indicative of the result of intermediate diagnostic relevance.

The method may further comprise transmitting said signal to a remote recipient with a wireless communication unit of the device. This signal may contain the result of intermediate diagnostic relevance, e.g. to facilitate a diagnosis by the remote recipient of the information, e.g. a remote medical practitioner.

The signal may further contain identification information of a diagnostic test and/or a global position of the user of such a diagnostic test.

### BRIEF DESCRIPTION OF THE DRAWINGS

Embodiments of the invention are described in more detail and by way of non-limiting examples with reference to the accompanying drawings, wherein:
FIG. 1 schematically depicts a device including a touchscreen;
FIG. 2 schematically depicts a system diagram of such a device;
FIG. 3 depicts a flow chart of a computer-implemented method according to an embodiment
FIG. 4 schematically depicts a diagnostic test delivery device;
FIG. 5 schematically depicts a diagnostic test method according to an embodiment; and
FIG. 6 depicts a flow chart of a computer-implemented method according to another embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

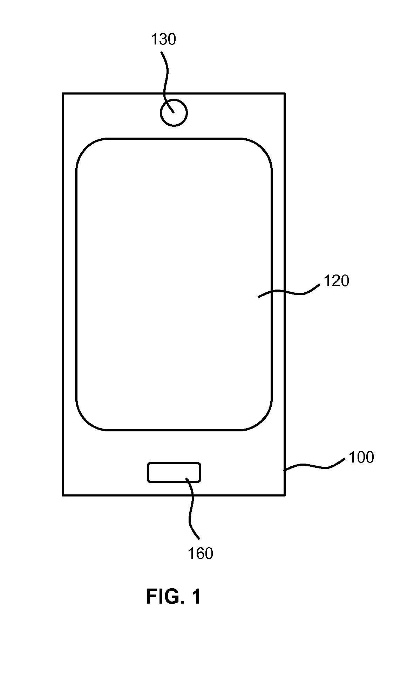

FIG. 1 schematically depicts a device 100 that may be used for implementing various aspects of the present invention. The device 100 typically comprises a touchscreen 120 and may further comprise a camera 130 that faces the same direction as the touchscreen 120, e.g. a front camera, and a user interface 160, which may take any suitable shape, e.g. one or more buttons, a trackball, a touch pad, a keyboard and so on, or any suitable combination thereof. The device 100 may be a device dedicated to evaluating skin protrusions or may be a multi-purpose device. In a preferred embodiment, the device 100 is an ubiquitous multi-purpose portable device such as a smart phone, a tablet, a personal digital assistant or the like. In an embodiment, the touchscreen 120 is a touch-sensitive display screen.

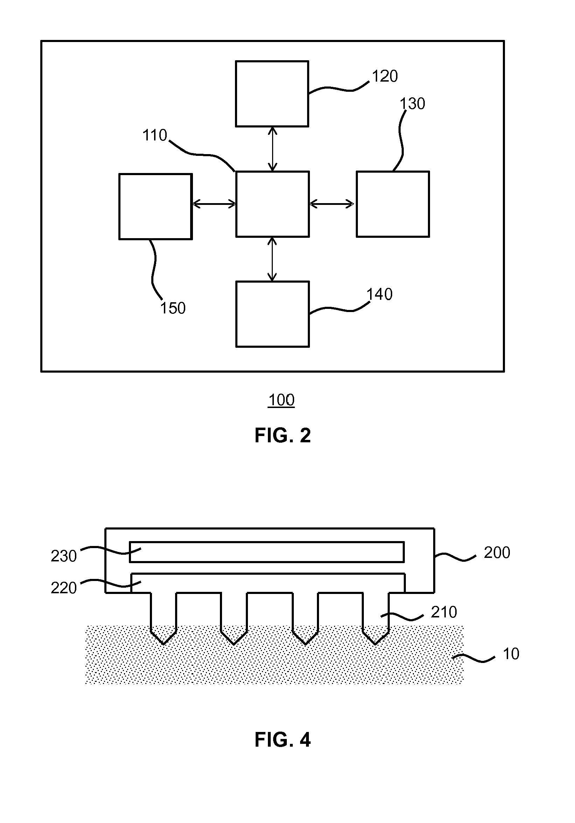

FIG. 2 schematically depicts a relevant part of the system architecture of such a device 100. The device 100 typically comprises a central processor 110, which may be a single processor or a cluster of cooperating (sub-)processors, e.g. a cluster of cores interacting to implement the processor functionality. Any suitable processor arrangement may be contemplated. The processor 110 is arranged to control the touchscreen 120, that is to receive signals from the touchscreen 120 and interpret these signals, e.g. to identify user instructions provided by the touchscreen 120. This may be implemented in any suitable manner, e.g. by identification of the activation of a particular set of touchscreen pixels or by detection of a pressure applied to a region of the touchscreen, e.g. a cluster of touchscreen pixels and translate this detected pressure into a particular user command. The processor 110 may interact with the touchscreen 120 in any suitable manner; this is well-known per se and will therefore not be explained in further detail for the sake of brevity only. In case of the touchscreen 120 being a touch-sensitive display screen, the processor 110 may further control the display screen by providing the display screen with drive signals for the display screen.

The processor 110 further controls the camera 130 if present, for instance in response to the touchscreen 120 or to the user interface 160, by activating the camera upon a user indicating that a picture or video should be taken with the camera 130 through the touchscreen 120 or user interface 160. Again, this is well-known per se and will therefore not be explained in further detail for the sake of brevity only.

The processor 110 may further control at least one wireless communication module 140 to control communications from the device 100 to a remote component and optionally to control communication from a remote component to the device 100. Any suitable wireless communication protocol may be used for any of the wireless communication between device 100 and such remote components, e.g., an infrared link, Zigbee, Bluetooth, a wireless local area network protocol such as in accordance with the IEEE 802.11 standards, a 2G, 3G or 4G telecommunication protocol, and so on. The at least one wireless communication module 140 may communicate with the remote component through any suitable relay component, e.g. a wireless modem, base station, mobile telecommunication mast, satellite, and so on. The at least one wireless communication module 140 may further be adapted to access a remote data source such as the Internet, for instance to retrieve data from a remote database as will be explained in more detail below.

The processor 110 may further control a global positioning module 150 for determining the global position of the device 100, e.g. through obtaining time-of-flight information from satellites having known geostationary positions, or through obtaining any other suitable signals that allow the determination of the global position of the device 100 through well-known triangulation methods. In an embodiment, the global positioning module 150 may be at least in part implemented in software for execution on the processor 110.

The above highlighted components of the device 100 have been highlighted as these components may be used in at least some of the embodiments of the present invention. It should however be understood that this is not be construed as these components being essential to the device 100 or to provide an exhaustive list of components comprised by the device 100; it will be apparent to the skilled person that the device 100 may comprise any suitable number and type of components.

FIG. 3 depicts a flow chart of a method 300 according to an embodiment, highlighting the principle on which the present invention is based. Specifically, the invention is based on the insight that a touchscreen 120 of a device 100 may be used to determine the size of a skin protrusion, e.g. a skin anomaly such as a rash or lump, or a skin bump resulting from the administration of a diagnostic test to a skin region using said region as an in-situ laboratory, e.g. a tuberculosis test as will be explained in more detail below.

The method 300 starts in step 310, e.g. by switching on the device 100 and activating a computer program product, e.g. an app or other software program on the device 100, that facilitates the evaluation of a skin protrusion with the touchscreen 120. Next, in step 320, the touchscreen 120 is brought into physical contact with a skin region containing the skin protrusion(s). The camera 130 may be used to generate guidance information with the processor 110, for instance by capturing an image of the skin region as the device 100 approaches the skin region and evaluating the captured image, e.g. using well-known object recognition algorithms, to identify the location of the skin protrusion relative to an area of the touchscreen 120 that is intended to make contact with the skin protrusion, e.g. a central region of the touchscreen 120. In case the processor 110 determines that the skin protrusion is misaligned with the intended contact area of the touchscreen 120, the processor 110 may generate guidance instructions, e.g. audible instructions on a speaker of the device 100, to correct for this misalignment.

The method 300 then proceeds to step 330 in which a touchscreen stimulus resulting from the contact between the skin protrusion and the touchscreen 120 is determined. This for instance may be the area size of the touchscreen activated by the contact with the skin protrusion, e.g. the number of contiguous pixels activated by this contact. This for instance provides an indication of the circumference of the skin protrusion, which indication may be used to extrapolate the size of the skin protrusion in step 340. Such an extrapolation for instance may rely on a predictable aspect ratio of the skin protrusion having a given circumference. In the context of the present application, the size of a skin protrusion typically comprises at least the height of the skin protrusion, i.e. the amount (as a length measurement) by which the skin is raised and/or the circumference (diameter) of the skin protrusion at the base, i.e. at the point where the protrusion protrudes from the skin.

In an embodiment, the touchscreen stimulus may include a pressure indication, i.e. the amount of pressure exerted on the touchscreen 120 by the skin protrusion. It is known per se that such pressure indications may be extracted from a touchscreen; this is for instance disclosed in European patent applications EP 2,620,844 and EP 2,390,772, as well as in US patent application US 2014/0028575 A1. The pressure indication may be a pressure difference (Δp) with the pressure exerted by remainder of the skin region in contact with the touchscreen 120, which has the advantage that the method is less sensitive to the absolute amount of pressure applied by the user of the device 100 when pressing the device 100 against the skin region including the skin protrusion, thus producing a more reliable skin protrusion size. The thus obtained pressure indication may be used to derive the size of the skin protrusion in step 340, either by using the pressure indication in isolation or by combining the aforementioned pixel area information and the pressure indication to obtain the size of the skin protrusion.

The processor 110 typically generates a signal indicative of the derived size of the skin protrusion, which may be a signal for further processing, a display signal for displaying skin protrusion size information on a touch-sensitive display screen 120, a data signal for storage in a data storage device such as a (remote) memory) and so on.

The method 300 preferably further comprises wirelessly communicating the signal indicative of the skin protrusion size to a remote component, e.g. a remote device operated by a medical professional such as a mobile communication device in a remote medical care centre such as a clinic or hospital, using the wireless communication module 140. The signal for instance may contain information pertaining to the skin protrusion size, which information therefore is of intermediate diagnostic relevance as it enables the remote medical practitioner to reach a diagnosis based on the provided information, e.g. a skin condition from which the patient is suffering, and to take the appropriate follow-up actions, e.g. communicating the diagnosis to the device 100, issuing a prescription for medication to the device 100 (as well as optionally to a dispensary in the local vicinity of the device 100) such that its user can collect the medication locally if possible, sending appropriate medication to the user of the device 100 and so on. In case the location of the device 100 and its user are a priori unknown, the signal communicated in step 350 may further comprise global position information of the device 100, which for instance may be obtained using the global positioning module 150 if present.

In an embodiment, the signal indicative of the skin protrusion size may be wirelessly communicated to the remote component in an encrypted form. This for instance enhances the security of the signal and protection of the data encompassed by the signal. Such encryption for instance may be required to comply with relevant data protection legislation, e.g. in a relevant jurisdiction and to comply with clinical governance arrangements throughout, e.g. in order to comply with data capture, storage, transfer and/or destruction provisions. The processor 110 may be adapted to encrypt the signal indicative of the skin protrusion size using any suitable encryption protocol. Alternatively, the device 100 may comprise an encryption module for encrypting the signal indicative of the skin protrusion size. The remote component is typically configured to decrypt the encrypted signal indicative of the skin protrusion size. The relevant encryption information, e.g. encryption key or the like, may be provided to the remote component in any suitable manner. This is well-known per se in the art and will not be explained in further detail for the sake of brevity only.

In an embodiment, the information pertaining to the skin protrusion size may comprise an automated evaluation of the skin protrusion size, e.g. in the case of an automated evaluation of a diagnostic test such as a tuberculosis (TB) test. In this embodiment, the processor 110 may be adapted to compare the determined skin protrusion size against a predefined threshold indicative of the patient having the condition for which has been tested, and to determine that the test is positive if the size of the skin protrusion meets or exceeds this predefined threshold. In this embodiment, the information pertaining to the skin protrusion size may comprise the automated diagnosis, which may be displayed on the touchscreen module 120 for the user of the device 100 to take follow-up actions and/or may be communicated to the remote component in optional step 350 as previously explained, e.g. for a medical practitioner to take the appropriate follow-up actions. The method 300 may subsequently terminate in step 360.

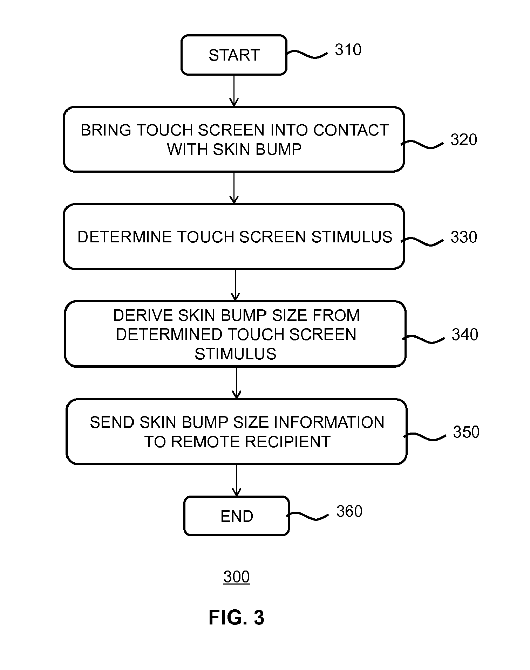

An example embodiment in which the method 300 forms part of a diagnostic test regime will be explained in further detail with the aid of FIG. 4-6. FIG. 4 schematically depicts a test delivery device 200 comprising a plurality of micro-needles 210 in fluid communication with a test fluid reservoir 220 and a delivery mechanism 230 for delivering the test fluid to a skin region 10 upon application of the test delivery device 200 to the skin region 10. The correct application of the test delivery device 200 to the skin region 10 typically requires the micro-needles 210 to pierce the skin region 10 for percutaneous delivery of the test fluid to the skin region 10. The test delivery device 200 may take any suitable shape, e.g. an adhesive patch for adhesion to the skin region 10 to ensure that the test delivery device 200 is in contact with the skin region 10 for long enough to ensure complete (percutaneous) delivery of the test fluid to the skin region 10. The test delivery device 200 may have any suitable dimension, e.g. a length and width each in the range of 10-30 mm by way of non-limiting example.

The test delivery device 200 may deliver any diagnostic test to the skin region 10 that triggers a skin reaction by forming a skin protrusion, the size of which is indicative of the diagnostic result. In an embodiment, the test delivery device 200 is for delivering a tuberculosis test. In this embodiment, the test fluid may comprise any suitable antigens for developing the desired skin reaction. An example of a well-known suitable test fluid for TB testing is based on purified protein derivative (PPD), which test fluid is for instance known as a Mantoux test fluid. This test fluid is however sensitive to heat-induced degradation and can yield false positives, e.g. due to cross-reactivity with the Bacillus Calmette-Guerin (BCG) vaccine, such that this test fluid is less preferred for TB testing, especially in hot climates where the test delivery device may need a certain shelf life. Recently, J. Jin et al. in Advanced Healthcare Materials, 2014(3), pages 349-353 have suggested the delivery of such a PPD-based TB test using a micro-needle based test delivery device 200.

More recently developed tests for TB testing are based on a combination of mycobacterium tuberculosis (Mtb) specific antigens to avoid cross-reactivity with the BCG vaccine and to improve diagnostic accuracy. An example of such a test is the Interferon Gamma Release Assay (IGRA) blood test. An skin-based administration version of this test is scheduled to be marketed by the Danish Statens Serum Institute:
http://www.ssi.dk/English/RandD/Research%20areas/Diagnostics/New%2 0tuberculosis%20skin%20test.aspx, as retrieved from the Internet on 31 March 2015.

The present inventors have realized that the micro-needle based test delivery device 200 is also suitable for delivery of a test serum based on a combination of mycobacterium tuberculosis (Mtb) specific antigens, such as early secretory antigenic target (ESAT), e.g. ESAT-6, and culture filtrate antigen (CFP), e.g. CFP-10 antigens. It is known that it is challenging to obtain a highly concentrated test fluid suitable for percutaneous administration using biological agents such as purified protein derivatives (PPD) which constitute the current mainstay of skin testing for TB. However, the inventors have surprisingly found that such problems are less prevalent when obtaining the high concentrations required for delivery through the test delivery device 200 using synthetic Mtb specific antigens.

According to an aspect, the test delivery device 200 may be provided as part of a diagnostic test kit that further comprises a computer program product of that implements one or more embodiments of the method of the present invention of the touchscreen-based evaluation of the skin reaction site to generate a result of intermediate diagnostic relevance, e.g. a result that may be used by a medical practitioner to reach a diagnosis or to generate a diagnosis from the touchscreen-based evaluation of the skin reaction site, as will be explained in more detail below. The computer program product may be provided on a computer-readable storage medium that is supplied together with the test delivery device 200, e.g. an RF tag or the like that may be read by the device 100, or may be provided by reference in the form of a link to a remote computer-readable storage medium, e.g. a remote server that may be contacted over the Internet or over a mobile telecommunications network, for downloading the computer program product to the device 100, e.g. in the form of an app or the like.

In an embodiment, the diagnostic test kit is for testing tuberculosis, in which case the test delivery device 200 preferably comprises a combination of Mtb-specific antigens as previously explained. However, it should be understood that the test delivery device 200 alternatively may comprise a PPD-based TB test or indeed any diagnostic test that may be applied to a region of the skin of a patient and for which the test result is derived from a reaction of the skin to the administered test.

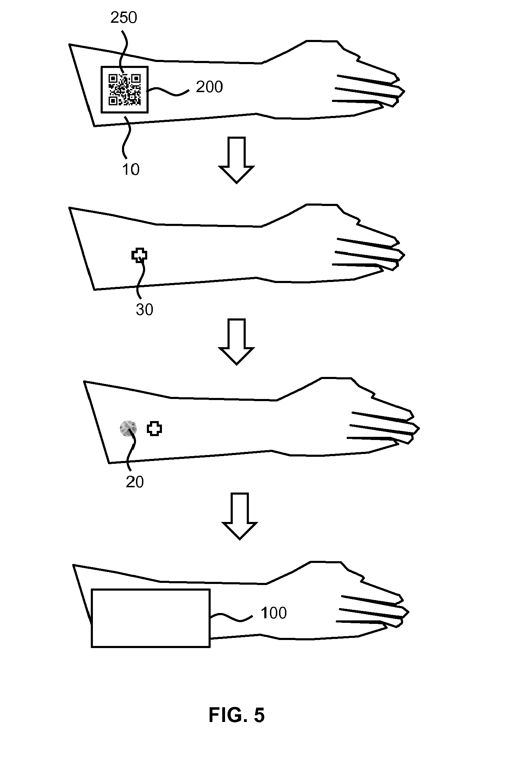

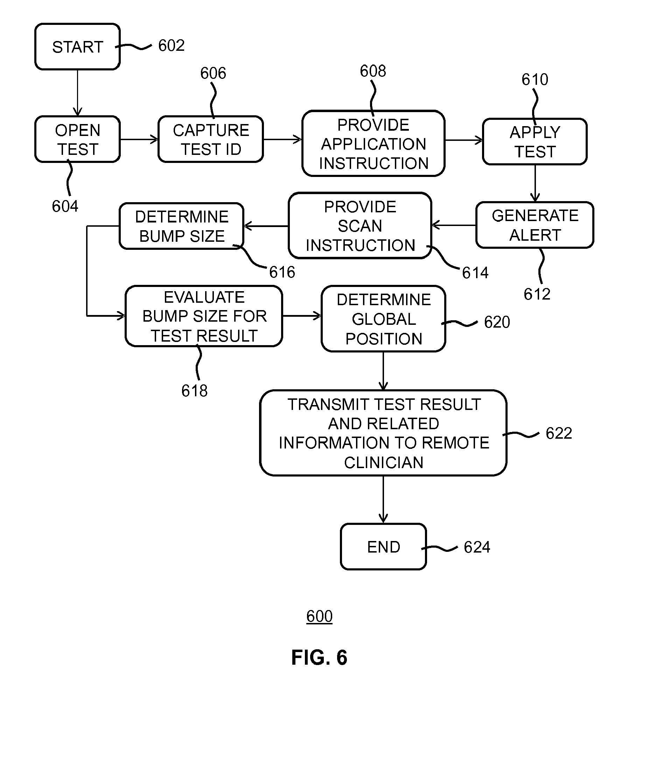

FIG. 5 schematically depicts an example method of how a diagnostic test using such a test delivery device 200 may be operated, whereas FIG. 6 depicts a flowchart of such a diagnostic test method using the aforementioned diagnostic test kit, wherein the computer program product is installed on a device 100, e.g. a mobile communications device. Both figures will be described simultaneously below. The method 600 starts in step 602, e.g. by activating the computer program product on the device 100, after which the method proceeds to step 604 in which the test delivery device 200 is opened, e.g. unwrapped. In an embodiment, the test delivery device 200 may comprise a computer-readable test identifier 250 such as a QR code, a bar code or the like, which may be exposed at the same time. The device 100 may be used to capture the test identifier 250, e.g. using camera 130, and to retrieve test application instructions for provision to the user in step 608. For example, the test application instructions may be embedded in the computer program and retrieved from memory into which the computer program loaded or may be retrieved from a remote source by connecting to the remote source using the wireless communication unit 140 and providing the test identifier to the remote source, e.g. a remote database, which remote source provides the appropriate test application instructions in return. The test application instructions may be provided to the user in step 608 in any suitable way, e.g. by displaying the instructions on a touch-sensitive display screen 120, by providing audible instructions on a loudspeaker of the device 100, and so on.

In an embodiment, the device 100 is further configured to capture the time at which the test ID is captured in step 606, as this time may be used to evaluate compliance of the user with the prescribed test protocol, as will be explained in further detail below. Alternatively, the user may be prompted to manually flag that the test has been correctly applied, e.g. by providing the device 100 with a particular user input, with the device 100 being configured to capture the time at which this particular user input is received.

In accordance with step 610, the user applies the test delivery device 200 to a region 10 of skin such as the forearm as shown in FIG. 5, for example in accordance with provided instructions. In an embodiment, correct application of the test delivery device 200 to the skin region 10 leaves a mark 30 on the skin, thus providing a visual check to the user allowing the user to verify if the test has been applied correctly. The instructions provided by the device 100 may prompt the user to check for the presence of this marker 30 and may instruct the user to reapply the test, e.g. by applying more pressure, in case the marker 30 cannot be observed.

After the correct application of the diagnostic test with the test delivery device 200, the test typically needs to develop for a defined period of time before a reliable test result can be extracted from the skin. In other words, the skin must be given enough time to react to the applied test fluid in order to reliably diagnose a positive test from a skin protrusion 20 formed in the region 10 as shown in FIG. 5. The user or test subject may be responsible for ensuring compliance with the appropriate test protocol before collecting the test result.

However, it is preferred that such compliance is monitored by the device 100, which compliance typically may be monitored by the computer program product provided with the diagnostic test kit, e.g. an app running on the device 100. This for instance may be achieved by generating an alert in step 612 prompting the user to collect the test result after sufficient time has elapsed from the device 100 collecting the timestamp indicating the application of the diagnostic test. This may be achieved in any suitable manner, for instance by the device monitoring the amount of elapsed time and comparing the monitored amount against a target amount associated with the sufficient development of the diagnostic test on the skin region 10, by the device calculating a point in time at which the diagnostic test has sufficiently developed on the skin region 10 and generating the alert once this point in time has been reached, and so on. The alert may be provided in any suitable form, e.g. as a visual alert on a touch-sensitive display screen 120 of the device 100, as an audible alert or a loudspeaker of the device 100, and so on.

Additionally or alternatively, the step 612 may generate an alert in case the user is attempting to prematurely generate the test result, which for instance may be detected by the user selecting a skin evaluation function in the computer program of the computer program product. This reduces the risk that the user prematurely evaluates the diagnostic test, and therefore reduces the risk of inaccurate test results, in particular false negatives.

Once the test has sufficiently developed, the user may be provided in step 614 with instructions on how to apply the touchscreen 120 of the device 100 to the skin region 10, as shown in the bottom drawing of FIG. 5. This may include audible guidance instructions to ensure that the skin protrusion 20 is brought into contact with an appropriate region of the touchscreen 120, e.g. a region sensitive enough to facilitate the determination of the size of the skin protrusion 20, e.g. a (near-)central region of the touchscreen 120. This is for instance relevant to touchscreens operating on an induced deformation of an upper screen layer, where the more central regions of the touchscreen may be more sensitive than the peripheral regions due to a greater ability to deform, i.e. such more central regions being more deformable or flexible. The camera 130 may assist in the generation of such guidance instructions by capturing one or more images of the skin region 10 to be contacted with the touchscreen 120 upon the device 100 approaching the skin region 10, wherein the processor 110 may be adapted to evaluate the captured image(s) to extract orientation information therefrom, for instance using object recognition algorithms that recognize the location of the skin protrusion 20 and/or the marker 30 relative to the touchscreen 120, which relative location may be used by the processor 110 to generate steering instructions, preferably in an audible form, to the user such that the user can guide the touchscreen 120 to the correct location on the skin region 10 to which the diagnostic test has been applied.

Once the touchscreen 120 has been brought into contact with the skin region 10, the size of the skin protrusion 20 is determined in step 616 from the touchscreen stimulus generated by the skin protrusion 20 as explained in more detail above. In case of a self-contained diagnostic test kit, the thus obtained skin protrusion size may be compared against a defined threshold in step 618, which defined threshold may be defined within the program code of the computer program product, to determine if the diagnostic test is positive. Such positive test is typically diagnosed if the skin protrusion size meets or exceeds such a predefined threshold, which then may indicate the reaction of the test fluid with biomarkers indicative of a medical condition, e.g. tuberculosis, in the body of the individual to which the test was applied. The diagnostic test result may be presented to the user in any suitable form, e.g. by displaying the diagnostic test result on a display such as a touch-sensitive display screen 120.

In an embodiment, the diagnostic test may further yield supplementary data such as colorimetric data for determining erythema in case of a TB test. To this end, the method 600 may further comprise capturing an image of the skin protrusion 20 with the camera 130, for instance when approaching the skin region 10 including the skin protrusion 20 with the device 100, and processing the image with the processor 110 to extract the degree of erythema from the captured image. Such supplementary data, e.g. erythema, blistering and breakdown of the integrity of the skin may be used as a guide to future management of the resulting test site. Severe reactions in the test site, e.g. demonstrated through erythema may require a dry occlusive dressing following removal of the adhesive test patch. The device 100 may generate instructions, e.g. audible or visual instructions, for applying such a dressing upon the detection of the supplementary data.

In an embodiment, the processor 110 further generates a signal for transmission to a remote component, e.g. a remote device, in step 622, which signal is indicative of the skin protrusion size derived from the touchscreen stimulus. This may be an indication of the diagnostic test result mentioned above. The signal may further include at least one of the test identifier 250 and an indication of the compliance of the applied test with recommended test protocols as previously explained. The remote component may be located in a remote medical facility operated by medical practitioners, which medical practitioners may take a further course of action upon receiving the signal from the device 100. For example, in case of receiving a signal of intermediate diagnostic relevance, the medical practitioner may reach a diagnosis based on this signal and take further actions if necessary, such as ordering further investigations, sending medical assistance and/or appropriate medication to the location of the device 100, e.g. medication to treat or manage the diagnosed medical condition. Where the signal already contains an indication of the diagnosis, the medical practitioner of course does not have to make the diagnosis anymore and may simply take the further actions.

As previously explained, the signal for transmission to a remote component may be encrypted prior to transmission for security reasons and/or governance reasons.

The user of the diagnostic test kit may have an address known to the medical practitioner, for instance because the user has previously registered his or her address with the medical practitioner or has registered his or her address when purchasing or otherwise receiving the diagnostic test kit. In such a scenario, any further actions considered appropriate by the medical practitioner may be directed to the registered address. The user may be identified from the identification code 250, for instance from a database in which the user is associated with the appropriate identification code 250.

However, in some scenarios the medical practitioner in the remote location may not have contact details for the user of the diagnostic test kit on record. This for instance may be the case in developing parts of the world where addresses are not being used and/or are not routinely available. In an embodiment, this problem may be addressed by the device 100 further comprising the global positioning unit 150, wherein in step 620 the global position of the device 100 is determined by the processor 110 controlling the global positioning unit 150, which global position information is included in the signal sent to the remote component in step 622, such that the medical practitioner may direct the appropriate further actions to the thus provided global position. The method 600 may subsequently terminate in step 624.

Aspects of the present invention may be embodied as a computer-implemented skin protrusion evaluation method. Aspects of the present invention may take the form of a computer program product embodied in one or more computer-readable medium(s) having computer readable program code embodied thereon. The computer readable program code is suitable for execution on a processor of a device further comprising a touchscreen under control of the processor. Aspects of the present invention may take the form of such a device including the computer program product or take the form of a test kit protect including a diagnostic test kit and the computer program product.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like. More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out the methods of the present invention by execution on the processor 110 may be written in any combination of one or more programming languages, including an object oriented programming language such as Java, Smalltalk, C++ or the like and conventional procedural programming languages, such as the "C" programming language or similar programming languages. The program code may execute entirely on the processor 110 as a stand-alone software package, e.g. an app, or may be executed partly on the processor 110 and partly on a remote server. In the latter scenario, the remote server may be connected to the head-mountable computing device 100 through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the processor 110 of the device 100, such that the instructions create means for implementing the functions/acts specified in the flowchart and/or block diagram block or blocks. These computer program instructions may also be stored in a computer-readable medium that can direct the device 100 to function in a particular manner.

The computer program instructions may be loaded onto the processor 110 to cause a series of operational steps to be performed on the processor 110, to produce a computer-implemented process such that the instructions which execute on the processor 110 provide processes for implementing the functions/acts specified in the flowchart and/or block diagram block or blocks.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A computer program product comprising a computer-readable medium carrying program code for, when executed on a processor (110) of a device (100) further comprising a touchscreen (120), implementing the steps of:
determining a touchscreen stimulus generated by a skin protrusion (20) in a skin region (10) brought into contact with the touchscreen;
processing the touchscreen stimulus to derive a size of said skin protrusion from the touchscreen stimulus; and
generating a signal indicative of said derived size.

2. The computer program product of claim 1, wherein said determining comprises determining at least one of an area of the touchscreen (120) in contact with the skin protrusion (20) and a pressure applied to the touchscreen by said skin protrusion.

3. The computer program product of claim 1 or 2, wherein the skin protrusion (20) is a reaction to a diagnostic test applied to the skin region, the program code further implementing the step of comparing the size of the skin protrusion against a predefined threshold to determine a test result, wherein the signal is indicative of said test result.

4. The computer program product of claim 3, wherein the device (100) further comprises a camera (130) under control of said processor (110), the program code further implementing the step of capturing an image of the skin protrusion (20) and extracting colorimetric information from the captured image to determine the test result.

5. The computer program product of claim 3 or 4, wherein the diagnostic test is a tuberculosis test.

6. The computer program product of any of claims 3-5, wherein the device (100) further comprises a camera (130) under control of said processor (110), the program code further implementing the step of capturing an identifier (250) of a test kit for administering the diagnostic test with said camera.

7. The computer program product of claim 6, the program code further implementing the steps of generating and providing test kit administration instructions to a user.

8. The computer program product of claim 6 or 7, wherein the step of capturing said identifier (250) is performed when administering the diagnostic test to the skin region, the program code further implementing the steps of:
determining a time period between capturing the identifier and determining the touchscreen stimulus; and
determining compliance with a recommended test regime from the determined time period.

9. The computer program product of any of claims 1-8, wherein the device (100) further comprises a wireless communication unit (140) under control of said processor (110), the program code further implementing the step of transmitting said signal to a remote recipient with the wireless communication unit.

10. The computer program product of claim 9, wherein said signal further includes the diagnostic test result and the test identifier.

11. The computer program product of any of claims 1-8, wherein the device (100) further comprises a global positioning unit (150) under control of said processor (110), the program code further implementing the step of determining a global position of the device with the global positioning unit upon determining the touchscreen stimulus and including the determined global position in the signal.

12. A device (100) comprising a touchscreen (120), a processor (110) configured to interpret touchscreen contact information and the computer program product of any of claims 1-11, wherein the processor is adapted to execute said program code.

13. A diagnostic test kit comprising a diagnostic test delivery device (200) and the computer program product of any of claims 1-11.

14. The diagnostic test kit of claim 13, wherein the diagnostic test delivery device (200) comprises a micro-needle device containing tuberculosis antigens for delivery to a skin region (10) of a user.

15. A computer-implemented method for execution by a processor (110) of a device (100) further comprising a touchscreen (120), the method comprising:
determining a touchscreen stimulus generated by a skin protrusion (20) in a skin region (10) brought into contact with the touchscreen;
processing the touchscreen stimulus to derive a size of said skin protrusion from the touchscreen stimulus; and
generating a signal indicative of said derived size.
